**Europäisches Patentamt**

**European Patent Office**

**Office européen des brevets**

(11) Veröffentlichungsnummer: **0 109 625**
**B1**

(12) **EUROPÄISCHE PATENTSCHRIFT**

(45) Veröffentlichungstag der Patentschrift:
**08.10.86**

(21) Anmeldenummer: **83111279.2**

(22) Anmeldetag: **11.11.83**

(51) Int. Cl.⁴: **C 07 D 307/60**

(54) Verfahren zur Herstellung von Dihydro-4,4-dimethyl-2,3-furandion.

(30) Priorität: **18.11.82 DE 3242560**

(43) Veröffentlichungstag der Anmeldung:
**30.05.84 Patentblatt 84/22**

(45) Bekanntmachung des Hinweises auf die Patenterteilung:
**08.10.86 Patentblatt 86/41**

(84) Benannte Vertragsstaaten:
**AT BE CH DE FR GB IT LI NL**

(56) Entgegenhaltungen:
**EP - A - 0 006 156**

**CHEMICAL ABSTRACTS, Band 92, Nr. 5, 4. Februar 1980, Seite 765, Nr. 41738q, Columbus, Ohio, US**
**HOUBEN-WEYL: METHODEN DER ORGANISCHEN CHEMIE, 4. Auflage, Band IV/1a, Oxidation Teil I, Seite 512, G. Thieme Verlag, Stuttgart, DE**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,**
**Carl-Bosch-Strasse 38, D-6700 Ludwigshafen (DE)**

(72) Erfinder: **Paust, Joachim, Dr., Ringstrasse 3,**
**D-6708 Neuhofen (DE)**
Erfinder: **Leininger, Hartmut, Dr.,**
**Dr.-Siebenpfeiffer-Strasse 22, D-6730 Neustadt (DE)**

## Beschreibung

Die Erfindung betrifft ein verbessertes Verfahren zur Herstellung von Dihydro-4,4-dimethyl-2,3-furandion durch Oxidation von Pantolacton mit Chlor und Calciumoxid in wasserfreiem Medium.

Dihydro-4,4-dimethyl-2,3-furandion, das auch unter dem Namen Ketopantolacton bekannt ist, stellt eine bedeutende Vorstufe zur Synthese von optisch aktivem Dihydro-3-hydroxy-4,4-dimethyl-2(3H)-furanon dar. Diese Verbindung, welche auch als Pantolacton bezeichnet wird, ist ein wichtiges Ausgangsmaterial für die Herstellung von R-(+)-Pantothensäure bzw. R-(+)-Panthenol.

Aus JP-A-79188257 ist es bekannt, dass Pantolacton (II) in einem inerten Lösungsmittel mit Brom in sehr guten Ausbeuten zu dem gewünschten Ketopantolacton (I) oxydiert werden kann. Nachteilig an diesem Verfahren ist, dass Oxidationen mit Brom in der Technik immer mit grossen Schwierigkeiten verbunden sind, da Reaktionen mit Brom emailierte Gefässe und Leitungen erfordern, welche sehr teuer sind, und ausserdem eine genaue Dosierung des Broms verfahrenstechnisch nicht einfach zu realisieren ist. Zudem ist Brom ein ausgesprochen teures Oxidationsmittel. Darüber hinaus sind die Raum-Zeit-Ausbeuten bei diesem Verfahren vergleichsweise niedrig und der Energieaufwand beträchtlich.

Weiterhin ist aus Houben-Weyl «Methoden der organischen Chemie» IV/1a, Seite 512, bekannt, dass Alkohole in Gegenwart von basischen Mitteln, wie Pyridin oder NaHCO$_3$, mit Halogen in wasserfreiem Lösungsmittel zu Carbonylverbindungen oxidiert werden können.

Aus EP-A-0006156 ist weiterhin bekannt, dass Ketopantolacton durch Oxidation von racemischem Pantolacton vorteilhaft unter Verwendung von festem Calcium-, Barium- oder Lithiumhypochlorit in organischer Phase hergestellt werden kann.

Die Verwendung der festen Hypochlorite als Oxidationsmittel, insbesondere Calciumhypochlorit, hat jedoch im technischen Massstab verschiedene Nachteile. Beispielsweise muss das Oxidationsmittel aufwendig durch Trocknung bei 60 bis 70 °C im Hochvakuum während 24 h vorbereitet werden. Das zum Einstz kommende Oxidationsmittel enthält Verunreinigungen, wie Calciumcarbonat und Natriumchlorid. Ferner ist zur Bestimmung des Calciumhypochlorit-Gehalts, der für die Einhaltung der Stöchiometrie der Reaktion bekannt sein muss, eine besondere Analytik notwendig. Nachteilig ist weiterhin die relativ lange Reaktionszeit. Schliesslich sind die in den beschriebenen Beispielen der EP-A-0006156 genannten Ausbeuten um 75% für ein technisches Verfahren nicht ausreichend.

Es wurde nun überraschenderweise gefunden, dass man Dihydro-4,4-dimethyl-2,3-furandion in wesentlich höheren Ausbeuten als bisher und erheblich einfacher durch Oxidation von Dihydro-3-hydroxy-4,4-dimethyl-2(3H)-furanon in einem organischen Lösungsmittel erhält, wenn man racemisches R,S-Pantolacton mit Chlor in organischer Phase vorlegt und gepulvertes Calciumoxid unter Ausschluss von Wasser kontinuierlich zudosiert.

Gegenstand der Erfindung ist daher ein Verfahren zur Herstellung von Dihydro-4,4-dimethyl-2,3-furandion durch Oxidation von racemischem Dihydro-3-hydroxy-4,4-dimethyl-2(3H)-furanon mit freien Halogenen in einem inerten organischen Lösungsmittel, das dadurch gekennzeichnet ist, dass man die Oxidation durch allmähliche Zugabe von gepulvertem Calciumoxid zu einer Lösung von racemischem Dihydro-3-hydroxy-4,4-dimethyl-2(3H)-furanon und Chlor in dem inerten organischen Lösungsmittel unter Ausschluss von Wasser bei Temperaturen von −10 bis 50 °C durchführt, wobei man, bezogen auf 1 Moläquivalent Dihydro-3-hydroxy-4,4-dimethyl-2(3H)-furanon, 1,2 bis 1,6 Moläquivalente Chlor und 2,0 bis 4,0 Moläquivalente Calciumoxid verwendet.

Es wird angenommen, dass die Oxidation nicht über die Stufe des Calciumhypochlorits, zumindest nicht über gebildetes festes Calciumhypochlorit oder Chlorkalk erfolgt, da waserfreies Calciumoxid mit trockenem Chlor in einem organischen Lösungsmittel nicht zu Calciumhypochlorit oder Chlorkalk reagiert. Offenbar werden zur Bildung von Calciumhypochlorit Calciumhydroxid und zumindest geringe Mengen Wasser benötigt, da Calciumhypochlorit technisch durch Einleiten von Chlor in eine wässrige Suspension von Calciumhydroxid und Aussalzen erhalten wird.

Deshalb ist der glatte Verlauf der erfindungsgemässen Oxidation überraschend sowie der Umstand, dass ausgezeichnete Ausbeuten um 90% und höher innerhalb der kurzen Reaktionszeit von 60 bis 120 min unter den milden Reaktionstemperaturen von 20 bis 30 °C erreicht werden können.

Die Umsetzung erfolgt in einem gegen Oxidation inerten Lösungsmittel. Geeignet sind beispielsweise halogenierte Kohlenwasserstoffe wie Methylenchlorid oder Chloroform, aromatische Kohlenwasserstoffe wie Benzol oder Chlorbenzol und insbesondere Acetonitril.

Die Oxidation wird bei Temperaturen von −10 bis 50 °C, vorzugsweise bei 20 bis 40 °C durchgeführt. Als Reaktionszeit wählt man im allgemeinen 0,3 bis 2,0 h. Bevorzugt wird eine Reaktionszeit von 1 bis 2 h.

Die Aufarbeitung des Reaktionsgemisches nach Filtration kann in an sich bekannter Weise durch Destillation oder Kristallisation nach Eindampfen erfolgen.

Besonders vorteilhaft ist die Umkristallisation des Ketopantolactons aus Methyl-tert-butylether.

### Beispiel 1

In einem Vierhalskolben, der mit einem mechanischen Rührer, Rückflusskühler, Innenthermometer und Gaseinleitungsrohr ausgestattet ist, wurden 32,5 g racemisches Dihydro-3-hydroxy-4,4-dimethyl-2(3H)-furanon (R,S-Pantolacton) in 300 ml über Molekularsieb getrocknetem Acetonitril vorgelegt.

In diese Lösung gaste man bei einer Temperatur von 0 bis 10 °C 25,0 g Chlor ein, das mittels Durch-

leiten durch eine mit konzentrierter Schwefelsäure gefüllte Waschflasche getrocknet worden war.

Nach Entfernen des Gaseinleitungsrohres gab man mit Hilfe eines Feststoffdosiertrichters 43,7 g Calciumoxid innerhalb von 60 min zur Reaktionsmischung. Durch Kühlung mit einem Kältebad wurde die Innentemperatur hierbei auf 25 °C gehalten. Nach beendeter Zugabe wurde 5 min nachgerührt.

Der Rückstand wurde darauf filtriert und mit 100 ml Acetonitril gewaschen und die klare, farblose Lösung am Rotationsverdampfer bei 14 mbar und Raumtemperatur eingeengt. Man erhielt 33,3 g farblose Kristallmasse mit Schmelzpunkt 63 bis 65 °C. Die Ausbeute an Dihydro-4,4-dimethyl-2,3-furandion wurde gaschromatographisch mit innerem Standard bestimmt. Sie beträgt 89% bei einem 4,4-%-Anteil an nicht umgesetztem Ausgangsmaterial Dihydro-3-hydroxy-4,4-dimethyl-2(3H)-furanon. Dies entspricht einem Gewichtsanteil von 28,5 g Dihydro-4,4-dimethyl-2,3-furandion.

Umkristallisation aus Methyl-tert-butylether ergab 24,0 g Ketopantolacton mit dem Schmelzpunkt 67 bis 68 °C.

Beispiel 2

Arbeitet man wie in Beispiel 1, jedoch bei einer Reaktionstemperatur von 30 °C anstelle von 25 °C, so erhielt man das Dihydro-4,4-dimethyl-2,3-furandion in einer Ausbeute von 91%.

Beispiel 3

Arbeitet man wie in Beispiel 1, gab jedoch das Calciumoxid innerhalb von 120 min anstelle von 60 min zur Reaktionsmischung, so erhielt man das Dihydro-4,4-dimethyl-2,3-furandion in einer Ausbeute von 95%.

## Patentansprüche

1. Verfahren zur Herstellung von Dihydro-4,4-dimethyl-2,3-furandion durch Oxidation von racemischen Dihydro-3-hydroxy-4,4-dimethyl-2(3H)-furanon mit freien Halogenen in einem inerten organischen Lösungsmittel, dadurch gekennzeichnet, dass man die Oxidation durch allmähliche Zugabe von gepulvertem Calciumoxid zu einer Lösung von racemischem Dihydro-3-hydroxy-4,4-dimethyl-2(3H-furanon und Chlor in dem inerten organischen Lösungsmittel unter Ausschluss von Wasser bei Temperaturen von −10 bis 50 °C durchführt, wobei man, bezogen auf 1 Moläquivalent Dihydro-3-hydroxy-4,4-dimethyl-2(3H)-furanon, 1,2 bis 1,6 Moläquivalente Chlor und 2,0 bis 4,0 Moläquivalente Calciumoxid verwendet.

2. Verfahren gemäss Anspruch 1, dadurch gekennzeichnet, dass man die Oxidation bei 20 bis 40 °C durchführt.

3. Verfahren gemäss Anspruch 1, dadurch gekennzeichnet, dass man die Oxidation in Acetonitril durchführt.

4. Verfahren gemäss Anspruch 1, dadurch gekennzeichnet, dass man das erhaltene Rohprodukt aus Methyl-tert.-butylether umkristallisiert.

## Claims

1. A process for the preparation of dihydro-4,4-dimethyl-furan-2,3-dione by oxidation of racemic dihydro-3-hydroxy-4,4-dimethyl-2(3H)-furanone with free halogens in an inert organic solvent, wherein the oxidation is carried out by gradually adding powdered calcium oxide to a solution of racemic dihydro-3-hydroxy-4,4-dimethyl-2(3H)-furanone and chlorine in the inert organic solvent in the absence of water at a temperature of from −10 to 50 °C, from 1.2 to 1.6 mole equivalents of chlorine and from 2.0 to 4.0 mole equivalents of calcium oxide being used per mole equivalent of dihydro-3-hydroxy-4,4-dimethyl-2(3H)-furanone.

2. A process as claimed in claim 1, wherein the oxidation is carried out at from 20 to 40 °C.

3. A process as claimed in claim 1, wherein the oxidation is carried out in acetonitrile.

4. A process as claimed in claim 1, wherein the crude product obtained is recrystallized from methyl tert.-butyl ether.

## Revendications

1. Procédé de préparation de dihydro-4,4-diméthyl-2,3-furanedione par l'oxydation de dihydro-3-hydroxy-4,4-diméthyl-2(3H)-furanone racémique avec des halogènes libres dans un solvant organique inerte, caractérisé en ce que l'on réalise l'oxydation par l'addition graduelle d'oxyde de calcium pulvérisé à une solution de dihydro-3-hydroxy-4,4-diméthyl-2(3H)-furanone racémique et de chlore dans un solvant organique inerte, sous exclusion d'eau, à des températures qui fluctuent de −10 à 50 °C, en utilisant par rapport à un équivalent molaire de dihydro-3-hydroxy-4,4-diméthyl-2(3H)-furanone, 1,2 à 1,6 équivalent molaire de chlore et 2,0 à 4,0 équivalents molaires d'oxyde de calcium.

2. Procédé suivant la revendication 1, caractérisé en ce que l'on réalise l'oxydation à une température qui varie de 20 à 40 °C.

3. Procédé suivant la revendication 1, caractérisé en ce que l'on réalise l'oxydation dans de l'acétonitrile.

4. Procédé suivant la revendication 1, caractérisé en ce que l'on recristallise le produit brut obtenu dans de l'éther méthyl-tert-butylique.